(19) Europäisches Patentamt
European Patent Office
Office européen des brevets

(11) Veröffentlichungsnummer : **0 015 237**
**B1**

(12) **EUROPÄISCHE PATENTSCHRIFT**

(45) Veröffentlichungstag der Patentschrift : **15.12.82**

(51) Int. Cl.³ : **C 08 F 16/26**, C 07 D211/46, C 08 L101/00// C08L29/10

(21) Anmeldenummer : **80810046.5**

(22) Anmeldetag : **08.02.80**

(54) **Homo- und Copolymerisate von Vinyläthern von Polyalkylpiperidinolen und deren Verwendung als Stabilisatoren für Kunststoffe.**

(30) Priorität : **14.02.79 CH 1465/79**

(43) Veröffentlichungstag der Anmeldung : **03.09.80 (Patentblatt 80/18)**

(45) Bekanntmachung des Hinweises auf die Patenterteilung : **15.12.82 Patentblatt 82/50**

(84) Benannte Vertragsstaaten : **CH DE FR GB IT**

(56) Entgegenhaltungen :
**DE A 2 352 658**
**GB A 1 394 952**

(73) Patentinhaber : **CIBA-GEIGY AG**
**Patentabteilung Postfach**
**CH-4002 Basel (CH)**

(72) Erfinder : **Nikles, Erwin, Dr.**
**Bodenackerstrasse 14**
**CH-4410 Liestal (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**EP 0 015 237 B1**

Homo- und Copolymerisate von Vinyläthern von Polyalkylpiperidinolen und deren Verwendung als Stabilisatoren für Kunststoffe

Die Erfindung betrifft Homo- und Copolymerisate von 4-Vinyloxypolyalkylpiperidinen, die als Stabilisatoren für Kunststoffe, insbesondere gegen deren Schädigung durch Licht, verwendet werden können. Die Erfindung betrifft ferner die den Polymerisaten zugrundeliegenden monomeren Vinyläther, die ebenfalls als Lichtschutzmittel verwendbar sind.

Es ist bekannt, dass Derivate von Polyalkylpiperidinen, insbesondere Derivate des 2,2,6,6-Tetramethylpiperidins, ausgezeichnete Lichtschutzmittel für Kunststoffe sind. Ein Beispiel hierfür ist das Bis-(2,2,6,6-tetramethyl-4-piperidyl) sebacat, das vor allem als Lichtschutzmittel für Polyolefine industrielle Verwendung findet. Weitere Ester und Aether von Polyalkyl-4-piperidinolen sind in den DE-OS 1 929 928 und 2 258 752 beschrieben. Für bestimmte Anwendungsgebiete ist die Migrationstendenz und die Extrahierbarkeit solcher niedermolekularer Piperidinderivate zu hoch. Dies ist insbesondere bei der Anwendung in dünnen Schichten der Fall, beispielsweise in Filmen, Ueberzügen oder Fasern.

Es wurden daher bereits auch höhermolekulare, insbesondere polymere Derivate von Polyalkylpiperidinen als Lichtschutzmittel für Kunststoffe vorgeschlagen, beispielsweise Polykondensations- und Polyadditionsprodukte in der DE-OS 2 719 131 oder Polymerisate von ungesättigten Estern und Amiden in der EP-Anm. 78 100 360.3. Ein allgemeines Problem bei solchen polymeren Lichtschutzmitteln ist deren Verträglichkeit mit den zu schützenden Kunststoffen. Im allgemeinen sinkt zwar mit steigendem Molekulargewicht des Stabilisators sein Migrationsvermögen und seine Extrahierbarkeit, andrerseits sinkt aber auch seine Verträglichkeit mit dem polymeren Substrat. Die Verträglichkeit ist aber nicht nur vom Molekulargewicht des Stabilisators abhängig, sondern auch von seiner chemischen Struktur sowie von der Art des Substrates.

Es wurde gefunden, dass bei der Polymerisation von Vinyläthern von Polyalkyl-4-piperidinolen Produkte entstehen, die mit vielen Klassen von Kunststoffen gut verträglich sind und dazu eine ausgezeichnete Lichtschutzwirkung zeigen, die auch bei längerem Gebrauch des Kunststoffes erhalten bleibt. Die Eigenschaften dieser Polymerisate lassen sich ferner modifizieren durch Copolymerisation mit anderen Monomeren, soweit diese zur Copolymerisation befähigt sind.

Gegenstand der Erfindung sind daher Homo- und Copolymerisate von Vinyläthern der Formel I,

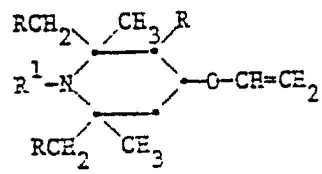

(I)

worin

R Wasserstoff oder $CH_3$ ist und $R^1$ Wasserstoff, $C_1$-$C_8$-Alkyl, Benzyl, Allyl, Formyl oder Acetyl bedeutet. Bevorzugt ist R Wasserstoff.

$R^1$ als Alkyl kann dabei z.B. Methyl, Aethyl, Propyl, Butyl, Isoamyl, Hexyl, n-Octyl oder iso-Octyl sein, bevorzugt ist $R^1$ Methyl.

Bevorzugt sind Homo- und Copolymerisate von Vinyläthern der Formel I, worin $R^1$ Methyl und worin R Wasserstoff ist.

Bevorzugt sind ferner die Homopolymerisate von Vinyläthern der Formel I, insbesondere von 1,2,2,6,6-Pentamethyl-4-piperidyl-vinyläther.

Die Homopolymerisation der Vinyläther der Formel I kann nach den für Vinyläther allgemein bekannten Methoden geschehen, wie sie beispielsweise in Houben-Weyl, Methoden der Organischen Chemie, Bd. 14/1, S. 927-956, beschrieben sind. Dies schliesst sowohl die radikalische als auch metallorganische oder kationische Polymerisation ein. Die radikalische Polymerisation wird durch UV-Bestrahlung oder durch radikalbildende Katalyzatoren initiert, wie z.B. Peroxyverbindungen, und führt nur zur Polymerisaten niedrigen Molekulargewichts. Die Polymerisation mit metallorganischen Mischkatalysatoren, wie z.B. solchen aus Aluminiumalkylen und Titanhalogeniden, ermöglicht die Herstellung stereospezifischer Polymerisate. Von besonderer Bedeutung ist die kationische Polymerisation der Vinyläther, die durch elektrophile Katalysatoren, wie z.B. Borfluorid, Phosphorpentafluorid, Aluminiumchlorid, Zinntetrachlorid, initiiert wird. Besonders wirksam als Initiator für die Polymerisation der Verbindungen der Formel I sind Borfluorid und seine EDA-Komplexe, wie z.B. $BF_3$-Aetherate oder $BF_3$-Aminkomplexe.

Unabhängig vom gewählten Initiatorsystem kann die Polymerisation in Substanz, in Lösung, in Dispersion oder Emulsion geschehen, vorwiegend arbeitet man in Lösung oder in Substanz.

Die Polymerisation verläuft bei Raumtemperatur oder sogar bei Teuperaturen unterhalb derselben (bis zu − 30 °C) in ausreichender Geschwindigkeit. In einzelnen Fällen, beispielsweise in starker Verdünnung, kann jedoch eine Beschleunigung der Polymerisation durch Erwärmen angebracht sein. Das Molekulargewicht der Polymerisate lässt sich durch die Menge des Initiators, durch die Geschwindigkeit der Initiator-Zugabe oder durch Zusatz eines Reglers oder eines Kettenabbrechers regeln.

Die erfindungsgemässen Homopolymerisate der Vinyläther der Formel I haben im allgemeinen ein mittleres Molekulargewicht von etwa 600 bis 100 000, was etwa einem Polymerisationsgrad von 3 bis 500 entspricht. Für die Verwendung als Stabilisatoren in Kunststoffen sind vor allem Polymerisate mit einem mittleren Molekulargewicht von etwa 3 000 bis 50 000 von Interesse, da in diesem Bereich sowohl eine gute Migrations- und Extraktionsbeständigkeit als auch eine ausreichende Verträglichkeit im Substrat gewährleistet ist.

Wie bereits erwähnt, können die Eigenschaften dieser polymeren Stabilisatoren durch Copolymerisation mit einem zur Copolymerisation befähigten Comonomeren modifiziert werden. Als solche eignen sich in erster Linie äthylenisch ungesättigte Verbindungen wie Alkyl-vinyläther, z.B. Methyl-vinyläther oder Isobutyl-vinyläther ; Vinylester, wie z.B. Vinylacetat oder -propionat ; andere Vinylverbindungen, wie z.B. Styrol, N-Vinylpyrrolidon, Vinylchlorid oder Vinylidenchlorid ; Derivate von Acryl- oder Methacrylsäure, wie z.B. Methylacrylat, tert. Butylacrylat, Methylmethacrylat, Acrylamid, N-Butylmethacrylamid, Acrylnitril oder Methacrylnitril, sowie Maleinsäurederivate wie Ester, Amide und cyclische Imide der Maleinsäure. Bevorzugt sind unter den Copolymerisaten diejenigen, bei denen das Comonomere ein Vinyläther mit maximal 20 C-Atomen ist.

Die Menge an Comonomer kann bis zu 90 Mol-% betragen. Es sind auch höhere Comonomeranteile möglich, jedoch sinkt mit steigendem Comonomerengehalt die Wirksamkeit der Copolymeren als Lichtschutzmittel. Bevorzugt sind daher Copolymere mit einem Comonomerengehalt bis zu 50 Mol-%.

Die Copolymeren können statistische Copolymere, Block-copolymere oder alternierende Copolymere sein. Es hängt von der Art des verwendeten Initiators, des verwendeten Comonomers sowie von der Reihenfolge der Zugabe und sonstigen Verfahrensmassnahmen ab, welcher dieser Strukturtypen vorwiegend entsteht. Für die Copolymerisation mit anderen Vinyläthern eignen sich kationische Initiatoren, die Copolymerisation mit anderen ungesättigten Verbindungen kann sowohl kationisch wie radikalisch initiiert werden. Im allgemeinen gelten auch bei der Copolymerisation von Vinyläthern der Formel I die für die Copolymerisation von Vinyläthern allgemein bekannten Gesetzmässigkeiten, wie sie z.B. in Houben-Weyl, Methoden der Organischen Chemie, *14/1*, 956-972 beschrieben sind.

Die monomeren Vinyläther der Formel I sind neue Verbindungen, die ebenfalls Gegenstand der Erfindung sind. Ihre Herstellung kann durch die für die Herstellung von Vinyläthern allgemein bekannten Methoden geschehen. Hierzu gehört vor allem die Herstellung durch Addition von Acetylen an Polyalkyl-4-piperidinole in Gegenwart von Katalysatoren, die vorzugsweise unter Druck (etwa 10-20 atm.) ausgeführt werden kann. Als Katalysatoren eignen sich hierbei dieselben wie bei der Vinylierung einfacher Alkohole, wie sie z.B. von W. Reppe in Ann. *601,* 81 (1956) beschrieben wurden. Beispiele hierfür sind Kalium- oder Natriumhydroxid.

Ein anderes Verfahren, das sich vor allem als Labormethode eignet, ist die Umvinylierung von Polyalkyl-4-piperidinolen mit Vinylestern, wie z.B. Vinylacetat, oder mit Vinyläthern, wie z.B. Isobutylvinyläther. In beiden Fällen wird in Gegenwart von Schwermetallkatalysatoren, wie z.B. Quecksilbersalzen, und mit einem Ueberschuss an Vinylierungsmitteln gearbeitet.

Andere Methoden zur Herstellung der erfindungsgemässen Vinyläther sind die Umsetzung der Alkalisalze von Polyalkyl-4-piperidinolen mit Vinylchlorid oder die Pyrolyse von Acetalen von Polyalkyl-4-piperidinolen.

Die Vinyläther der Formel I sind flüssige oder niedrig schmelzende Verbindungen, die durch Destillation gereinigt werden können. Sie sind in den meisten organischen Lösungsmitteln gut löslich, in Wasser ist ihre Löslichkeit gering.

Die Vinyläther der Formel I besitzen ebenfalls eine Wirkung als Lichtschutzmittel und können als solche gebraucht werden. Wichtiger ist jedoch ihre Bedeutung als monomere Ausgangsmaterialien für die vorhin beschriebenen Homo- und Copolymerisate.

Beispiele für Vinyläther der Formel I, die sich zur Homo- und Copolymerisation eignen, sind :

2,2,6,6-Tetramethyl-4-piperidyl-vinyläther
1,2,2,6,6-Pentamethyl-4-piperidyl-vinyläther
2,3,6-Trimethyl-2,6-diäthyl-4-piperidyl-vinyläther
1-Propyl-2,2,6,6-tetramethyl-4-piperidyl-vinyläther
1-Isobutyl-2,2,6,6-tetramethyl-4-piperidyl-vinyläther
1-Hexyl-2,2,6,6-tetramethyl-4-piperidyl-vinyläther
1-Octyl-2,2,6,6-tetramethyl-4-piperidyl-vinyläther
1-Benzyl-2,2,6,6-tetramethyl-4-piperidyl-vinyläther
1-Allyl-2,2,6,6-tetramethyl-4-piperidyl-vinyläther
1-Benzyl-2,2,6,6-tetramethyl-4-piperidyl-vinyläther.

Die Homo- und Copolymerisate von Vinyläthern der Formel I können erfindungsgemäss als Stabilisatoren für Kunststoffe gegen thermisch-oxydativen, vorzugsweise gegen photochemischen Abbau verwendet werden. Als Kunststoffe kommen vor allem solche in Frage, die gegen Lichteinwirkung empfindlich sind, und wie sie beispielsweise in der DE-OS 26 47 452 auf Seite 12-14 aufgeführt sind.

Von besonderer Bedeutung ist die Stabilisierung von Polyolefinen, Styropolymerisaten und Polyurethanen. Beispiele hierfür sind Polyäthylen hoher und niedriger Dichte, Polypropylen, Aethylen-Propylen-Copolymerisate, Polystyrol, Styrol-Butadien-Acrylnitril-Copolymerisate, Mischungen von Polyolefinen oder von Styrolpolymerisaten, Polyurethane auf Polyäther- oder Polyesterbasis in Form von

Lacken, Fasern, Elastomeren oder Schaumstoffen.

Die erfindungsgemässen Polyvinyläther eignen sich in besonderem Masse für den Lichtschutz von latexierten und bei erhöhter Temperatur (z.B. 120 °C) behandelten Polypropylen-Multifilamenten.

Die erfindungsgemässen Stabilisatoren werden den Kunststoffen in einer Konzentration von 0,01 bis 5 Gew.-%, berechnet auf das zu stabilisierende Material, zugesetzt. Vorzugsweise werden 0,1 bis 2 Gew.-% der Verbindungen, berechnet auf das zu stabilisierende Material, in dieses eingearbeitet.

Die Einarbeitung kann nach der Polymerisation erfolgen, beispielsweise durch Einmischen der Verbindungen und gegebenenfalls weitere Additive in die Schmelze nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels.

Die Stabilisatoren können auch in Form eines Masterbatches, der diese Verbindungen beispielsweise in einer Konzentration von 2,5 bis 25 Gew.-% enthält, den zu stabilisierenden Kunststoffen zugesetzt werden.

Im Falle von vernetztem Polyäthylen werden die Verbindungen vor der Vernetzung beigefügt.

Ausser den polymeren Stabilisatoren können den Kunststoffen auch noch andere, bekannte Stabilisatoren zugesetzt werden. Dies können z.B. Antioxydantien, Lichtschutzmittel oder Metalldesaktivatoren sein, oder auch Costabilisatoren wie z.B. solche vom Typ der Phosphorigsäureester. Weiterhin können sonstige in der Kunststofftechnologie übliche Zusätze, wie z.B. Flammschutzmittel, Antistatika, Weichmacher, Gleitmittel, Treibmittel, Pigmente, Verstärkungsstoffe oder Füllstoffe zugesetzt werden.

Bei der Mitverwendung von bekannten Stabilisatoren können synergistische Effekte auftreten, was besonders bei Mitverwendung von anderen Lichtschutzmitteln oder von organischen Phosphiten häufig der Fall ist.

Die Erfindung betrifft daher auch die durch Zuzatz von 0,01 bis 5 Gew.-% eines Homo- oder Copolymerisates von einem Vinyläther der Formel I stabilisierten Kunststoffe, die gegebenenfalls noch andere bekannte und übliche Zusätze enthalten können. Die so stabilisierten Kunststoffe können in verschiedenster Form angewendet werden, z.B. als Folien, Fasern, Bändchen, Profile oder als Bindemittel für Lacke, Klebemittel oder Kitte.

Die Herstellung und Verwendung der erfindungsgemässen Verbindungen wird in den folgenden Beispielen näher beschrieben. Teile bedeuten darin Gewichtsteile und % Gewichtsprozente. Die Temperaturen sind in Celsius-Graden angegeben.

## Beispiel 1

1,2,2,6,6-Pentamethyl-4-piperidyl-vinyläther

Die Mischung von 28 g 1,2,2,6,6-Pentamethyl-4-piperidinol und 164 g Butyl-vinyläther wurde mit 2 g Quecksilberacetat versetzt und unter Rückfluss gekocht. Nach 11 Stunden wurde gaschromatographisch eine ca. 77 %-ige Umsetzung des eingesetzten Alkohols festgestellt. Der Ansatz wurde auf 600 ml Wasser gegossen und mit Hexan verdünnt. Die organische Phase wurde abgetrennt und mit wasserfreiem Natriumsulfat getrocknet. Das Lösungsmittel und der überschüssige Butyl-vinyläther wurden durch Destillation zurückgewonnen. Der Rückstand wurde bei 15 mm Hg destilliert, wobei 20 g 1,2,2,6,6-Pentamethyl-4-piperidylvinyläther vom Sdp. 98° erhalten wurden.

Dasselbe Produkt konnte auch durch Umsetzung von 1,2,2,6,6-Pentamethyl-4-piperidinol mit Acetylen bei 170° und 15 atm. Druck in Gegenwart von 2 g Kaliumhydroxyd gewonnen werden.

Analog wie oben beschrieben, wurde auch der 1-n-Octyl-2,2,6,6-tetramethyl-4-piperidyl-vinyläther hergestellt. (Reinigung durch Kurzwegdestillation bei 100°/0.01 mm Hg).

## Beispiel 2

1-Benzyl-2,2,6,6-tetramethyl-4-piperidyl-vinyläther

Die Mischung von 123,6 g 1-Benzyl-2,2,6,6-tetramethyl-4-piperidinol und 4 g Kaliumhydroxyd wurde durch azeotrope Destillation mit Toluol entwässert. Das Toluol wurde abgedampft und der Rückstand bei 0,01 mm Hg und 40° getrocknet. Anschliessend wurde diese Mischung bei 170-180° und 15 atm. Druck in einer Acetylenatmosphäre gerührt bis kein Acetylen mehr aufgenommen wurde (5 Stunden). Das Produkt wurde destilliert. Sdp. 116°/0.5 mm/Hg. Ausbeute 106 g.

In ähnlicher Weise wurden erhalten :

2,3,6-Trimethyl-2,6-diäthyl-4-piperidyl-vinyläther,
Sdp. 121°/18 mm Hg
1-Allyl-2,2,6,6-tetramethyl-4-piperidyl-vinyläther,
Sdp. 63°/0,1 mm Hg
1-Butyl-2,2,6,6-tetramethyl-4-piperidyl-vinyläther,

Sdp. 66-67°/0,1 mm Hg
2,2,6,6-Tetramethyl-4-piperidyl-vinyläther,
Sdp. 78°/19 mm Hg

Beispiel 3

1-Acetyl-2,2,6,6-tetramethyl-4-piperidyl-vinyläther

40 g 2,2,6,6-Tetramethyl-4-piperidyl-vinyläther wurden in 270 ml trockenem Pyridin gelöst und mit 200 ml Essigsäureanhydrid versetzt. Die Mischung wurde langsam erwärmt und schliesslich 36 Std. unter Rückfluss gekocht. Die flüchtigen Teile wurden im Vakuum einer Wasserstrahlpumpe auf dem siedenden Wasserbad abgedampft und der Rückstand im Hochvakuum destilliert. Sdp. 88°/0,1 mm Hg.

Beispiel 4

Poly(1,2,2,6,6-pentamethyl-4-piperidyl-vinyläther)

a) 50 g 1,2,2,6,6-Pentamethyl-4-piperidyl-vinyläther wurden tropfenweise bei − 20° in 3 Stunden mit 4 ml Bortrifluorid-Aethylätherat, gelöst in 4 ml trockenem Aether versetzt. Die Mischung wurde ca. 20 Stunden bei − 18° stehen gelassen. Das Produkt wurde durch Verdünnen mit Acetonitril gefällt, in Hexan gelöst, mit verdünnter Sodalösung gewaschen und wieder durch Zugabe von Acetonitril gefällt.
Smp. 128-134°. Mittleres Molekulargewicht $\bar{M}n$ : 27 000.

b) 100 g 1,2,2,6,6-Pentamethyl-4-piperidyl-vinyläther wurden in 100 ml trockenem Hexan gelöst und im Verlaufe von 6 Std. mit 15 ml Bortrifluorid-äthylätherat, gelöst in 30 ml trockenem Aether versetzt. Die hochviskos werdende Lösung wurde 3 Std. nach Beginn der Reaktion mit 50 ml Hexan und nach weitern 3 1/2 Std. mit 100 ml Methylenchlorid verdünnt. Nach insgesamt 9 1/2 Std. wurde das Gemisch mit 500 ml Methylenchlorid verdünnt, viermal mit je 100 ml 2-n Sodalösung und dann mit Wasser gewaschen, über wasserfreiem Natriumsulfat getrocknet, filtriert und eingedampft. Der flüssige Rückstand wurde in Gegenwart von insgesamt 2 Liter Acetonitril verrieben. Das sich fest abscheidende Produkt wurde abfiltriert und getrocknet. Man erhielt 88 g des Poly(1,2,2,6,6-pentamethyl-4-piperidyl-vinyläthers) vom Smp. 107-128°, $\bar{M}n$ : 5 900.

In analoger Weise wurden hergestellt :
Poly(1-allyl-2,2,6,6-tetramethyl-4-piperidyl-vinyläther),
Smp. 122-138°, $\bar{M}n$ : 27 400
Poly(1-butyl-2,2,6,6-tetramethyl-4-piperidyl-vinyläther),
Smp. 110-168°, $\bar{M}n$ : ca. 3 000-4 000.
Poly(1-acetyl-2,2,6,6-tetramethyl-4-piperidyl-vinyläther),
(Polymerisation in Methylenchlorid bei − 20° während 3 Tagen).

Beispiel 5

Poly(1-benzyl-2,2,6,6-tetramethyl-4-piperidyl-vinyläther)

5 g 1-Benzyl-2,2,6,6-tetramethyl-4-piperidyl-vinyläther wurden in 50 ml trockenem Toluol gelöst und mit 0,3 ml Bortrifluorid-äthylätherat, gelöst in 10 ml Toluol, versetzt. Nach 20-stündigem Stehen bei Raumtemperatur war die Lösung hochviskos. Die Mischung wurde mit 50 ml Methylenchlorid verdünnt, mit Sodalösung gewaschen, über wasserfreiem Natriumsulfat getrocknet und eingedampft. Der Rückstand, ein hartes Harz, wurde in Methyläthylketon gelöst und mit Methanol gefällt. Es wurden 4,2 g Polymeres mit einem Schmelzbereich 110-120° und einem mittleren Molekulargewicht $\bar{M}n$ von 60 300 erhalten.

Beispiel 6

Copolymerisation von 1,2,2,6,6-Pentamethyl-4-piperidylvinyläther mit Isobutyl-vinyläther

15 g 1,2,2,6,6-Pentamethyl-4-piperidyl-vinyläther und 7,6 g Isobutylvinyläther wurden in 20 ml Hexan gelöst und bei − 20° in 5 Stunden tropfenweise mit 1,2 ml Bortrifluorid-äthylätherat, gelöst in 10 ml trockenem Aether versetzt. Nach ca. 20 Stunden bei ca. − 20° wurde die Mischung mit Methylenchlorid verdünnt, mit verdünnter Sodalösung und Wasser gewaschen, getrocknet, filtriert und eingedampft. Das zurückbleibende Harz wurde mit 300 ml Acetonitril verrieben und 14 Stunden stehen gelassen. Das gebildete Festprodukt wurde abfiltriert und getrocknet. Smp. 72-83°, $\bar{M}n$ : 7 300, Stickstoffgehalt : 5,25 %.

In analoger Weise wurden 5 g 1,2,2,6,6-Pentamethyl-4-piperidylvinyläther und 1,83 g Aethylvinyläther in 10 ml Hexan copolymerisiert. Das erhaltene Copolymerisat schmilzt bei 86-93° und besitzt ein mittleres Molekulargewicht Mn von 6 300.

Beispiel 7

Copolymerisat aus 1,2,2,6,6-Pentamethyl-4-piperidylvinyläther und Acrylnitril

6 g 1,2,2,6,6-Pentamethyl-4-piperidyl-vinyläther und 6 g Acrylnitril wurden in 10 ml Toluol gelöst, mit 0,2 g Azoisobutyronitril versetzt und 14 Tage bei Raumtemperatur stehen gelassen. Die Mischung wurde mit Methylenchlorid verdünnt, mit Wasser gewaschen, getrocknet, filtriert und eingedampft. Der Rückstand wurde aus Hexan bei – 20° gefällt. Das erhaltene Produkt erweicht über 100° und schmilzt bis 260° nicht vollständig. $\bar{M}n$ ca. 2 500, Stickstoffgehalt 14,5 %.

Beispiel 8

Copolymerisat aus 2,2,6,6-Tetramethyl-4-piperidyl-vinyläther und N-Butyl-maleinimid

Die Lösung von 4 g 2,2,6,6-Tetramethyl-4-piperidyl-vinyläther, 3,3 g N-n-Butylmaleinimid und 50 mg Azoisobutyronitril in 40 ml Ligroin wurden unter Stickstoff 24 Stunden bei 50° gehalten. Das Lösungsmittel wurde abgedampft. Der Rückstand wurde mit Hexan behandelt und das feste Produkt abfiltriert und getrocknet. Smp.~125°, $\bar{M}n$ : 1 100, Stickstoffgehalt 7,9 %.

Beispiel 9

Lichtschutzwirkung in PP-Fasern

1 000 Teile unstabilisiertes Polypropylenpulver (Schmelzindex ~18) werden in einem Schnellmischer mit 1 Teil Calziumstearat, 0,5 Teilen Calzium-bis(4'-Hydroxy-3',5'-ditertiär-butyl-benzyl-äthylphosphonat), 2,5 Teilen Titandioxid und mit 3 Teilen der in der Tabelle angeführten Lichtschutzmittel gemischt und anschliessend in einem Extruder bei 220 °C extrudiert und granuliert. Das erhaltene Granulat wird in einer Labor-Schmelzspinnanlage bei einer maximalen Temperatur von 270 °C und einer Geschwindigkeit von 600 m/Minute zu einem 403/37 denier Multifilament versponnen. Dieses wird verstreckt und gezwirnt mittels einer Streckzwirnmaschine. Das Streckverhältnis ist 1: 3,2, sodass schliesslich Multifilamente 130/37 denier erhalten werden. Diese Multifilamente werden auf weissen Karten montiert, wobei an den Enden des Kartons je ein weiterer Kartonstreifen aufgeheftet wird, so dass die Fäden im mittleren Teil über 5 cm berührungsfrei aufgespannt sind. Die Fasern werden im Xenotest 1 200 belichtet. Als Mass der Schutzwirkung gilt die Belichtungszeit bis 50 % Reissfestigkeitsverlust. Die Ergebnisse sind in der Tabelle zusammengefasst.

| Verwendetes Lichtschutzmittel | Belichtungszeit bis 50 % Festigkeitsverlust |
|---|---|
| ohne | 400 h |
| 0,3 % Poly(1,2,2,6,6-pentamethyl-4-piperidyl-vinyläther) | 4 700 h |
| 0,3 % Poly(1-allyl-2,2,6,6-tetramethyl-4-piperidyl-vinyläther) | 1 175 h |

**Ansprüche**

1. Homo- und Copolymerisate von Vinyläthern der Formel I,

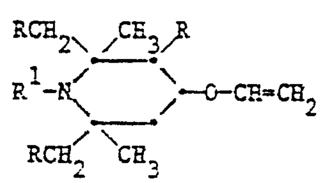

(I)

worin R Wasserstoff oder Methyl ist und $R^1$ Wasserstoff, $C_1$-$C_8$-Alkyl, Benzyl, Allyl, Formyl oder Acetyl bedeutet.

2. Homo- und Copolymerisate gemäss Anspruch 1 von Vinyläthern der Formel I, worin R Wasserstoff ist.

3. Homo- und Copolymerisate gemäss Anspruch 1 von Vinyläthern der Formel I, worin R Wasserstoff und $R^1$ Methyl ist.

4. Homopolymerisate gemäss Anspruch 1 von Vinyläthern der Formel I.

5. Poly(1,2,2,6,6-pentamethyl-4-piperidyl-vinyläther).

6. Copolymerisate gemäss Anspruch 1, worin das Comonomer ein Vinyläther mit max. 20 C-Atomen ist.

7. Verwendung von Homo- und Copolymerisaten von Vinyläthern der Formel I des Anspruchs 1 als Stabilisatoren für Kunststoffe gegen deren thermisch-oxydative und photochemische Schädigung.

8. Verwendung von Poly(1,2,2,6,6-pentamethyl-4-piperidyl-vinyläther) gemäß Anspruch 7.

9. Gegen thermisch-oxydative und photochemische Schädigung stabilisierter Kunststoff, dadurch gekennzeichnet, dass er 0,01 bis 5 Gew.-% eines Homo- oder Copolymerisates eines Vinyläthers der Formel I des Anspruchs 1, bezogen auf das zu stabilisierende Material, enthält.

10. Stabilisierter Kunststoff gemäss Anspruch 9, dadurch gekennzeichnet, dass er 0,01 bis 5 Gew.-% Poly(1,2,2,6,6-pentamethyl-4-piperidyl-vinyläther), bezogen auf das zu stabilisierende Material, enthält.

11. Stabilisierter Kunststoff gemäss Anspruch 9, dadurch gekennzeichnet, dass es sich bei dem zu stabilisierenden Material um ein Polyolefin, ein Styrolpolymerisat oder ein Polyurethan handelt.

12. Stabilisierter Kunststoff gemäss Anspruch 9, dadurch gekennzeichnet, dass er ausser dem erfindungsgemässen Stabilisator noch einen oder mehrere übliche Zusätze enthält.

13. Vinyläther der Formel I,

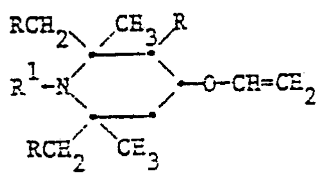

(I)

worin R Wasserstoff oder $CH_3$ ist und $R^1$ Wasserstoff, $C_1$-$C_8$-Alkyl, Benzyl, Allyl, Formyl oder Acetyl bedeutet.

## Claims

1. A homopolymer or copolymer of a vinyl ether of the formula I

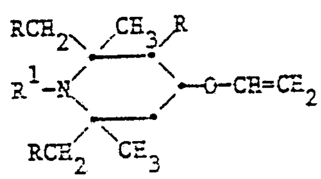

(I)

in which R is hydrogen or methyl and $R^1$ is hydrogen, $C_1$-$C_8$-alkyl, benzyl, allyl, formyl or acetyl.

2. A homopolymer or copolymer according to claim 1 of a vinyl ether of the formula I in which R is hydrogen.

3. A homopolymer or copolymer according to claim 1 of a vinyl ether of the formula I in which R is hydrogen and $R^1$ is methyl.

4. A homopolymer according to claim 1 of a vinyl ether of the formula I.

5. Poly-(1,2,2,6,6-pentamethyl-4-piperidyl vinyl ether).

6. A copolymer according to claim 1, in which the comonomer is a vinyl ether having not more than 20 C atoms.

7. The use of a homopolymer or copolymer of a vinyl ether of the formula I according to claim 1 as a stabiliser for plastics to protect them against thermo-oxidative and photochemical damage.

8. The use of poly-(1,2,2,6,6-pentamethyl-4-piperidyl vinyl ether) according to claim 7.

9. A plastic stabilised against thermo-oxidative and photochemical damage, which contains 0.01 to 5 % by weight, relative to the material to be stabilised, of a homopolymer or copolymer of a vinyl ether of the formula I of claim 1.

10. A stabilised plastic according to claim 9, which contains 0.01 to 5 % by weight, relative to the material to be stabilised, of poly-(1,2,2,6,6-pentamethyl-4-piperidyl vinyl ether).

11. A stabilised plastic according to claim 9, which is a polyolefin, a styrene polymer or a polyurethane.

12. A stabilised plastic according to claim 9, which in addition to the stabiliser according to the invention also contains one or more customary additives.

13. A vinyl ether of the formula I

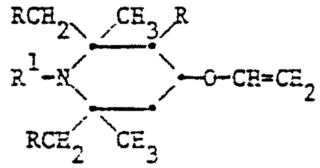

(I)

in which R is hydrogen or $CH_3$ and $R^1$ is hydrogen, $C_1$-$C_8$-alkyl, benzyl, allyl, formyl or acetyl.

**Revendications**

1. Homopolymères et copolymères d'éthers vinyliques répondant à la formule I

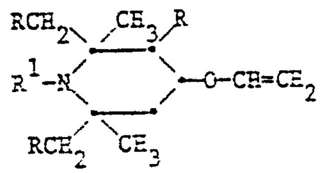

(I)

dans laquelle R représente l'hydrogène ou un méthyle et $R^1$ représente l'hydrogène, un alkyle en $C_1$-$C_8$, un benzyle, un allyle, un formyle ou un acétyle.

2. Homopolymères et copolymères selon la revendication 1, qui dérivent d'éthers vinyliques de formule I dans lesquels R représente l'hydrogène.

3. Homopolymères et copolymères selon la revendication 1, qui dérivent d'éthers vinyliques de formule I dans lesquels R représente l'hydrogène et $R^1$ un radical méthyle.

4. Homopolymères selon la revendication 1, qui dérivent d'éthers vinyliques de formule I.

5. Poly-(oxyde de vinyle et de pentaméthyl-1,2,2,6,6 pipéridyle-4).

6. Copolymères selon la revendication 1, dans lesquels le comonomère est un éther vinylique contenant au plus 20 atomes de carbone.

7. Application d'homopolymères et de copolymères d'éthers vinyliques de formule I selon la revendication 1 comme stabilisants pour des matières plastiques contre leur dégradation thermo-oxydative et photochimique.

8. Application du poly-(oxyde de vinyle et de pentaméthyl-1,2,2,6,6 pipéridyle-4) selon la revendication 7.

9. Matière plastique stabilisée contre la dégradation thermo-oxydative et photo-chimique, caractérisée en ce qu'elle contient de 0,01 à 5 % en poids d'un homopolymère ou copolymère d'un éther vinylique de formule I selon la revendication 1, par rapport à la matière à stabiliser.

10. Matière plastique stabilisée selon la revendication 9, caractérisée en ce qu'elle contient de 0,01 à 5 % en poids de poly-(oxyde de vinyle et de pentaméthyl-1,2,2,6,6 pipéridyle-4), par rapport à la matière à stabiliser.

11. Matière plastique stabilisée selon la revendication 9, caractérisée en ce que la matière à stabiliser est une polyoléfine, un polymère du styrène ou un polyuréthanne.

12. Matière plastique stabilisée selon la revendication 9, caractérisée en ce qu'elle contient, en plus du stabilisant conforme à l'invention, un ou plusieurs additifs usuels.

13. Ethers vinyliques répondant à la formule I

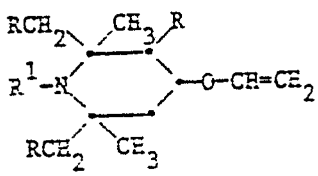

(I)

dans laquelle R représente l'hydrogène ou $CH_3$ et $R^1$ représente l'hydrogène, un alkyle en $C_1$-$C_8$, un benzyle, un allyle, un formyle ou un acétyle.